# EUROPEAN PATENT SPECIFICATION

(11) **EP 3 397 089 B1**
(45) Date of publication and mention of the grant of the patent: **19.02.2020**
(21) Application number: 16810482.6
(22) Date of filing: 06.12.2016
(51) Int. Cl.: A24F 13/02, A24F 13/14, A24F 47/00

(54) **HOLDER FOR AEROSOL GENERATING ARTICLE**
HALTER FÜR AEROSOLERZEUGENDEN ARTIKEL
SUPPORT POUR ARTICLE DE GÉNÉRATION D'AÉROSOL

(30) Priority: 29.12.2015 EP 15202954
(43) Date of publication of application: 07.11.2018
(73) Proprietor: Philip Morris Products S.A., 2000 Neuchatel (CH)
(72) Inventor: BATISTA, Rui, 1110 Morges (CH); ROJO-CALDERON, Noelia, 2000 Neuchatel (CH)
(74) Representative: Millburn, Julie Elizabeth
(86) International application number: PCT/IB2016/057391
(87) International publication number: WO 2017/115182

(56) References cited:
- DE-A1- 19 645 563
- FR-A1- 2 911 761
- US-A- 3 362 415
- US-A- 4 289 149
- US-A- 4 570 645

## Description

This invention relates to an aerosol generating article having a combustible heat source for heating an aerosol generating substrate.

A number of smoking articles in which tobacco is heated rather than combusted have been proposed in the art. An aim of such 'heated' smoking articles is to reduce certain smoke constituents of the type produced by the combustion and pyrolytic degradation of tobacco in conventional cigarettes.

In one known type of heated smoking article, an aerosol is generated by the transfer of heat from a combustible heat source to a physically separate aerosol generating substrate, for example containing tobacco. The aerosol generating substrate may be located within, around or downstream of the combustible heat source. During use, volatile compounds are released from the aerosol generating substrate by heat transfer from the combustible heat source and entrained in air drawn through the smoking article. As the released compounds cool, they condense to form an aerosol that is inhaled by the user.

Aerosol generating articles, for example heated smoking articles, may be configured so that the combustible heat source is blind, which may limit the amount or number volatile compounds released from combustion of the heat source that enter air drawn through the smoking article. Heated smoking articles having blind combustible heat sources may transfer heat to the aerosol-generating substrate primarily via conduction. Heated smoking articles may also be configured so that the heat source is non-blind. Heated smoking articles having non-blind combustible heat sources transfer heat to the aerosol-generating substrate primarily via convection through one or more air flow channels through the heat source, which allow volatile compounds from the heat source to enter air drawn through the smoking article. Because one aim of heated smoking articles is to reduce certain smoke constituents produced via combustion, heated smoking articles employing blind heat sources may be preferred.

Published PCT patent application, WO-A2-2009/022232, discloses an example of a heated smoking article having a non-blind heat source. The heated smoking article disclosed in WO-A2-2009/022232 comprises a combustible heat source, an aerosol generating substrate downstream of the combustible heat source, and a heat conducting element around and in contact with a rear portion of the combustible heat source and an adjacent front portion of the aerosol generating substrate. An air flow channel extends through the heat source such that air may be drawn through the channel downstream towards a mouthpiece.

Regardless of whether heated smoking articles include a blind or non-blind combustible heat source, the heat source may require direct contact with air to burn properly. Thus, heated smoking articles may be manufactured such that the heat source is exposed to a smoker and their surroundings during smoking. However, temperatures of the combustible heat sources may be quite high when burning. For example, temperatures of the heat sources during combustion may be more than 600ºC.

Published US patent, US 4,289,149, discloses a case for enclosing a cigarette while being smoked, in order to prevent ashes from scattering therefrom. The case includes an outer tube including a plurality of perforated openings around its side. A mouthpiece bit is fitted removably in one end of the outer tube. The bit includes a hole therethrough so as to allow drawing smoke therethrough. A wider end of the hole receives one end of a cigarette. An opposite end of the tube is fitted with a removable end cap which fits in one end of an inner tube fitted with spacer collars therearound that fit inside the outer tube. The inner tube also includes perforated openings therearound similarly to the openings in the outer tube. A space formed between the inner and outer tubes provides insulation therebetween, so that heat from the flame is not transmitted to the outer tube. A collar around an outer side of the inner tube serves to maintain the space a same distance all around the inner tube. A ring affixed on an inner side of the inner tube bear against the side of the cigarette so as to keep the flame from direct contact with the inner tube. A hole through a center of the end cap aligns with the center opening of the inner tube. A series of radial holes in the end cap communicate with the center opening of the inner tube.

One object of the invention is to reduce the temperature of an exposed element to which a user of an aerosol generating article having a combustible heat source or their environment may be exposed. Another object of examples of the invention is to maintain a blind heat source while reducing the temperature of an exposed element to which a user or their environment may be exposed.

The invention provides holder for an aerosol generating article according to claim 1. The holder comprises a mouthpiece having a mouth end and a distal end. The mouthpiece defines a passage extending from the mouth end to the distal end. The passage is configured to slidably receive the aerosol generating article inserted in a direction from the distal end towards the mouth end. The holder further comprises a cage extending from the mouthpiece and configured to surround at least a portion of a heat source of the aerosol generating article and configured to allow air to access the heat source. The cage is slidable about the mouthpiece. The mouthpiece defines a flange and the cage defines a stop configured to engage the flange as the cage is distally advanced over the mouthpiece. The holder may also optionally comprise a seal extending from an inner surface of the cage. The seal is arranged to surround and contact the aerosol generating article.

The invention further provides an assembly according to claim 7, comprising a holder and an aerosol generating article. The aerosol generating article comprises a heat source and an aerosol generating substrate downstream of the heat source and conductively coupled to the heat source such that heat from the heat source is transferred to the aerosol generating substrate without combusting the aerosol generating substrate. The aerosol generating article further comprises a wrapper circumscribing the aerosol generating substrate. The wrapper defines one or more ventilation through holes. The holder comprises a mouthpiece having a mouth end and a distal end and defining a passage extending from the mouth end to the distal end. The passage is configured to receive, for example slidably receive, the aerosol generating article inserted in a direction from the distal end towards the mouth end. The holder further comprises a cage configured to surround at least a portion of a heat source of the aerosol generating article. The holder comprises a seal extending from an inner surface of the cage. The seal is arranged to surround and contact the aerosol generating article downstream of the heat source when the aerosol generating article is received in the passage of the mouthpiece. The cage comprises a tubular wall defining one or more holes through the wall downstream of the seal. The assembly is configured such that drawing on the mouthpiece causes air to flow through the one or more holes of the tubular wall of the cage and through the one or more ventilation holes of the wrapper of the aerosol generating article.

The term "aerosol generating article" refers to an article comprising an aerosol generating substrate that releases volatile compounds to form an aerosol that may be inhaled by a user. The term "aerosol-generating substrate" refers to a substrate capable of releasing, upon heating, volatile compounds, which may form an aerosol. The aerosols generated from aerosol-generating substrates of articles according to the invention may be visible or invisible and may include vapours (for example, fine particles of substances, which are in a gaseous state, that are ordinarily liquid or solid at room temperature) as well as gases and liquid droplets of condensed vapours.

The terms "distal," "upstream," "proximal," and "downstream" are used to describe the relative positions of components, or portions of components, of an aerosol generating article. Aerosol generating articles according to the invention have a proximal end through which, in use, an aerosol exits the article for delivery to a user, and have an opposing distal end. The proximal end of the aerosol generating article may also be referred to as the mouth end. In use, a user draws on the proximal end of the aerosol generating article in order to inhale an aerosol generated by the aerosol generating article. The terms upstream and downstream are relative to the direction of aerosol movement through the aerosol generating article when a user draws on the proximal end.

Various aspects of the aerosol generating articles and holders according to the present invention may have one or more advantages relative to currently available aerosol generating articles that include a combustible heat source. For example, holders according to the invention provide a simple to use barrier to protect a smoker or their surrounding environment from contact with a combusted heat source having a high temperature. The end piece of the cage that surrounds the heat source may be separated from the heat source so that the temperature of the cage is lower than the temperature of the combusted heat source. The cage may also be configured to efficiently dissipate heat to reduce temperature of the cage relative to the combusted heat source. In some preferred embodiments, holders according to the invention are configured to prevent or reduce the number or amount volatile compounds released from the heat source during combustion from entering air drawn through the aerosol generating article and inhaled by a user. The holder may include a seal extending from an inner surface of the cage and arranged to surround and seal the aerosol generating article between the heat source and, for example, the aerosol-generating substrate to limit volatile compounds released from the heat source during combustion from entering drawn through the aerosol generating article. Additional advantages of one or more aspects of aerosol generating articles described herein will be evident to those of skill in the art upon reading and understanding the present disclosure.

The present invention relates to an aerosol generating article having a combustible heat source for heating an aerosol generating substrate and a holder for the aerosol generating article. The holder includes a cage configured to surround at least a portion of the heat source and to allow air to access the heat source. In use, the temperature of the cage is substantially lower than the heat source during combustion. Accordingly, the temperature of an element to which a user of the aerosol generating article may be exposed is reduced.

The holder comprises a mouthpiece having a mouth end and a distal end. The mouthpiece defines a passage for receiving, for example slidably receiving, an aerosol generating article having a combustible heat source. The passage is configured to receive the aerosol generating article inserted in a direction from the distal end towards the mouth end. The passage defines an inner surface of the mouthpiece. Preferably, at least a portion of the inner surface of the mouthpiece engages the aerosol generating article when the article is received in the passage. For example, the inner surface of the mouthpiece may define one or more detents or one or more reduced inner diameter portions that are configured to engage the aerosol generating article by interference fit. In addition or alternatively, the passage may be sized to engage the aerosol generating article along its length.

Alternatively, one or more additional elements disposed in the passage may engage the aerosol generating article. Regardless of whether the inner surface of the mouthpiece is configured to engage the aerosol generating article or whether an additional element in the passage is configured to engage the aerosol generating article, the aerosol generating article is preferably retained in a longitudinal position relative to the mouthpiece in use. The aerosol generating article is preferably insertable or removable from the passage with minimal force. For example, the aerosol generating article may be readily inserted or withdrawn from the passage by a user.

Preferably, the inner surface of the mouthpiece defines a stepped or tapered inner diameter such that the inner diameter in proximity to the mouth end is smaller than the outer diameter of the aerosol generating article to prevent advancement of the aerosol generating article in the passage beyond the mouth end. A portion of such a tapered inner surface may be configured to engage and retain the aerosol generating article.

The mouthpiece may be telescoping and may adapt a collapsed, ejection position and an expanded, use position. Preferably, the mouth end is movable distally relative to the distal end to the collapsed position to eject the aerosol generating article from the holder. The mouth end may be biased towards the expanded position so the mouth end moves distally relative to the distal end after a pushing force, for example to collapse the mouthpiece, is removed.

The mouthpiece may be formed of any suitable material or combination of materials. For example, the mouthpiece may be formed from a material comprising a polymer, such as a thermoplastic polymer, or a material comprising a metal, such as aluminium or stainless steel, or a ceramic material. The mouthpiece may for example be formed of wood or a carbon fibre or glass fibre material. Examples of suitable polymer materials that may be used to form the mouthpiece include polyetheretherketone (PEEK).

The cage and the mouthpiece may be formed from a single part. Alternatively, the cage and mouthpiece may be formed from separate parts. Preferably, the cage and mouthpiece are formed from separate parts.

Preferably, the cage is disposed, for example sildably disposed, about the mouthpiece, allowing the holder to have a more compact size when stored and an elongated size when in use. Preferably, the cage and mouthpiece are configured so that the cage is prevented from accidentally sliding off the mouthpiece. For example, the mouthpiece may comprise a distal flange that engages a proximal stop of, or attached to, the cage to prevent the cage from sliding distally off of the mouthpiece. The mouthpiece may also comprise, for example, a proximal flange or protrusion that engages the proximal stop to prevent the cage from sliding proximally off the mouthpiece.

The cage defines a bore having a longitudinal axis and an inner diameter greater than the outer diameter of the heat source of the aerosol generating article. In some examples, the bore diameter may be similar to that of the outer diameter of the aerosol generating article. The article may be held with a friction fit in the bore. A tapered opening or chamfer may be provided to facilitate insertion of the aerosol generating article. As used herein, the term 'diameter' denotes the maximum dimension in the transverse direction of the combustible heat source, aerosol generating article, holder or other apparatus or component. As used herein, the terms 'radial' and 'transverse' are used to describe the direction perpendicular to the longitudinal direction. In use, the cage at least partially surrounds the heat source. Preferably, the cage surrounds the heat source along the length of the heat source. The distance between the cage and the heat source, the material of the cage, the thickness of the cage, and the permeability of the cage, among other factors, may be selected to control the maximum temperature, relative to the heat source when in use. Preferably, the cage reaches a maximum temperature substantially lower than the heat source when the heat source is combusted and is disposed within the cage. For example, a temperature at an exposed surface of the cage may be at least 200ºC less than the temperature of a surface of the heat source. Preferably, a temperature at an exposed surface of the cage may be at least 300ºC less than the temperature of a surface of the heat source. For example, when the heat source temperature is about 400 ºC, preferably the exposed surface of the retainer is less than about 200 ºC, preferably less than about 150 °C.

The cage may be separated from the heat source by any suitable distance. For example, the radial clearance between the heat source and the cage may be between about 0.2 mm and about 3 mm, for example between 0.5 mm and 2 mm, or for example between 1 mm and 2 mm.

The cage may be made of any suitable material or combination of materials. Preferably, the material or materials forming the cage are heat resistant. For example, the cage may be formed from materials that can withstand temperatures of about 200 ºC or greater. Examples of suitable materials for forming the cage include polymers and metallic materials such as aluminium or stainless steel. In addition or alternatively, the cage may be formed from material comprising wood, carbon fibre or glass fibre. Examples of suitable polymers from which the cage or a portion of the cage may be made include polyethter ether ketone (PEEK), polyoxymethylene (POM), high density polyethylene (HDPE) and the like.

Preferably the material or materials from which the cage is made are sufficiently thermally conductive, sufficiently thin, or are of a sufficiently low density to rapidly dissipate heat.

The cage, or one or more portion of the cage, preferably has sufficient permeability to allow air to access the heat source through the cage to maintain combustion of the heat source. The term "permeability" refers to a percent of total area of a surface or a portion of a surface that is void space area. Preferably at least a portion of the cage that surrounds the heat source has sufficient permeability to allow a lighting of the heat source by a flame. Preferably the heat source can be lit through the cage. Preferably, the cage comprises a distal face defining an opening that has a cross sectional area that is the same as the bore of the cage. In addition or alternatively, the cage comprises openings, such as through-holes, disposed radially about the heat source. In preferred embodiments, the cage comprises a generally tubular wall with ventilation openings through the wall. In some preferred embodiments, the permeability of the portion of the cage surrounding the heat source is sufficient to maintain combustion of the heat source only when the ventilation openings or through holes are not covered. Accordingly, the openings may be covered to extinguish the heat source.

The cage may comprise a cover to aid in extinguishing the heat source. For example, the cover may comprise a sleeve that is movable from a first position to a second position. In the first position the sleeve does not cover the openings of the cage. In the second position the sleeve covers the openings and may aid in extinguishing the heat source. By way of another example, the cover may comprise a ring element disposed about a body of the cage and rotatable about a longitudinal axis of the cage. The ring may comprise through-holes that align with openings through the body of the cage when appropriately rotated. Rotation of the ring about the cage away from the aligned position may completely or partially block the openings through the body of the cage to aid in extinguishing the heat source.

Preferably, the cage comprises ventilation through-holes downstream of the portion surrounding the heat source. Preferably, such holes allow cool air to be drawn through the aerosol generating article. In preferred embodiments, the cage comprises a non-permeable wall between the distal end of the cage and the downstream ventilation openings. Such a wall may reduce the amount or number of combustion products from the heat source from entering the air drawn into the aerosol generating article.

The holder according to the present invention comprises a seal that extends from the inner surface of the cage into the bore of the cage. The seal is arranged to surround and contact the aerosol generating article downstream of the heat source. The seal may comprise an o-ring. The seal may prevent or limit combustion products released from the heat source from entering air drawn through the aerosol generating article. The seal is preferably positioned upstream of the downstream ventilation openings of the cage.

The seal may have any suitable inner diameter. Preferably, the inner diameter of the seal, in a relaxed state, is less than the outer diameter of the aerosol generating article. As an aerosol generating article is introduced into an end piece according to the present invention, the seal may deflect to allow passage of the article through the seal. The seal may serve to hold the cage in a longitudinal position relative to the aerosol generating article when the article is fully inserted into the end piece.

The seal may be integrally formed with the cage or attached to the cage in any suitable manner. The seal may be formed of any suitable material or combination of materials. Because the seal is configured to be placed in proximity to the heat source, the seal preferably comprises heat resistant materials. The seal preferably comprises a resilient material. Examples of materials that may be used to form a seal or a portion thereof include plastic materials and elastomers, for example nitrile or fluorocarbons (viton) materials.

A holder according to the present invention is configured to allow an aerosol generating article to be inserted, mouth end first, into a passage defined by the mouthpiece until the mouth end engages a shoulder or tapered portion in proximity to the mouth end. The cage may be distally advanced over the mouthpiece until the cage engages the mouthpiece and further advancement is prevented. Distal movement of the cage may occur until, for example, a proximal stop of the cage engages a distal flange of the mouthpiece. Openings at the distal portion of the cage allow for lighting of the heat source, continued combustion of the heat source, and evacuation of combustion gasses when the aerosol generating article is fully inserted into the holder. Openings in the cage permit outside air to be drawn into the aerosol generating article, through or around a heated aerosol generating substrate and into a user's mouth. Preferably, the openings that permit outside air to be drawn through the aerosol generating article are separated from more distal cage openings by a seal.

If the cage comprises a cover, the cover may be moved to cover distal openings in the cage to aid in extinguishing the heat source. If the mouthpiece is telescoping, the mouth end can be pressed and moved distally relative to the distal end of the mouthpiece to eject the extinguished smoking article.

The cage may include a flexible portion that permits a user to depress the flexible portion to pinch off the heat source when the user is done using the aerosol generating article. A system of the invention may include an extractor to, for example, push or pull the article to facilitate removal of the heat source. The extractor may be a separate element or may be integrally formed with one or more elements of the article.

Holders according to the present invention may be used with any suitable aerosol generating article having a combustible heat source. The aerosol generating article includes an aerosol generating substrate that may be heated by the combustible heat source to release one or more volatile compounds from the aerosol generating substrate.

An aerosol generating article for use with an end piece according to the present invention may include any suitable combustible heat source.

The combustible heat source is preferably a blind combustible heat source. As used herein, the term 'blind' describes a heat source that does not comprise any air flow channels that provide inhalation air to the aerosol generating substrate. In a blind combustible heat source, heat transfer from the blind combustible heat source to the aerosol-generating substrate occurs primarily by conduction and heating of the aerosol generating substrate by forced convection is minimized or reduced. The lack of any airflow channels through the blind combustible heat source advantageously substantially prevents or inhibits activation of combustion of the blind combustible heat source during puffing by a user. This substantially prevents or inhibits spikes in the temperature of the aerosol generating substrate during puffing by a user. By preventing or inhibiting activation of combustion of the blind combustible heat source, and so preventing or inhibiting excess temperature increases in the aerosol generating substrate, combustion or pyrolysis of the aerosol generating substrate under intense puffing regimes may be advantageously avoided. In addition, the impact of a user's puffing regime on the composition of the mainstream aerosol may be advantageously minimized or reduced. The inclusion of a blind combustible heat source may also advantageously substantially prevent or inhibit combustion and decomposition products and other materials formed during ignition and combustion of the blind combustible heat source from entering air drawn through the aerosol generating article during use thereof.

Alternatively, the combustible heat source comprises at least one longitudinal airflow channel, which provides one or more inhalation airflow pathways through the heat source to the aerosol generating substrate. This inhalation airflow channel may extend along the length of the heat source through which air may be drawn through the aerosol generating article for inhalation by a user. Such heat sources including one or more longitudinal inhalation airflow channels are referred to herein as "non-blind" heat sources.

The combustible heat source is preferably a carbonaceous heat source having a carbon content of at least about 35 percent, more preferably of at least about 40 percent, most preferably of at least about 45 percent by dry weight of the combustible heat source. Where the combustible heat source is a carbonaceous heat source, the combustible heat source may be formed from one or more suitable carbon-containing materials. The term "carbonaceous" refers to a material that comprises carbon.

The combustible heat source may be a combustible carbon-based heat source having a carbon content of at least about 50 percent. For example, the combustible heat source may be a combustible carbon-based heat source having a carbon content of at least about 60 percent, or at least about 70 percent, or at least about 80 percent by dry weight of the combustible heat source. The term "carbon-based" refers to a material comprises primarily of carbon or at least about 50% carbon, by dry weight of material.

One or more binders may be combined with the one or more carbon-containing materials to form the carbonaceous heat source. The combustible heat source may comprise one or more organic binders, one or more inorganic binders or a combination of one or more organic binders and one or more inorganic binders.

Instead of, or in addition to one or more binders, the combustible heat source may comprise one or more additives in order to improve the properties of the combustible heat source. Suitable additives include, but are not limited to, additives to promote consolidation of the combustible heat source (for example, sintering aids), additives to promote ignition of the combustible heat source (for example, oxidisers such as perchlorates, chlorates, nitrates, peroxides, permanganates, zirconium and combinations thereof), additives to promote combustion of the combustible heat source (for example, potassium and potassium salts, such as potassium citrate) and additives to promote decomposition of one or more gases produced by combustion of the combustible heat source (for example catalysts, such as CuO, Fe₂O₃ and Al₂O₃). Combustible heat sources for aerosol generating articles and methods for producing such heat sources are known in the art and described in, for example, US-A-5,040,552 and US-A-5,595,577.

Preferably, the combustible heat source has an apparent density of between about 0.8 g/cm³ and about 1.1 g/cm³. Preferably, the combustible heat source has a mass of between about 300 mg and about 500 mg, more preferably of between about 400 mg and about 450 mg. Preferably, the combustible heat source has a length of between about 7 mm and about 17 mm, more preferably of between about 7 mm and about 15 mm, most preferably of between about 7 mm and about 13 mm. Preferably, combustible heat sources according to the invention have a diameter of between about 5 mm and about 9 mm, more preferably of between about 7 mm and about 8 mm.

Preferably, the combustible heat source is of substantially uniform diameter. However, the combustible heat source may alternatively be tapered such that the diameter of one of the front end face and the rear end face of the combustible heat source is greater than the diameter of the other of the front end face and the rear end face thereof. For example, combustible heat sources may be tapered such that the diameter of the rear end face of the combustible heat source is greater that the diameter of the front end face of the combustible heat source. Preferably, the combustible heat source is substantially cylindrical. The combustible heat source may be a cylindrical combustible heat source of substantially circular cross-section or of substantially elliptical cross-section. In particularly preferred embodiments, the combustible heat source is a substantially cylindrical combustible heat source of substantially circular cross-section.

An aerosol generating article for use with a holder according to the invention may include any aerosol generating substrate.

Preferably, the aerosol generating substrate comprises at least one aerosol-former and a material capable of releasing volatile compounds in response to heating. The aerosol generating substrate may comprise other additives and ingredients including, but not limited to, humectants, flavorants, binders and mixtures thereof. Preferably, the aerosol generating substrate comprises nicotine. More preferably, the aerosol generating substrate comprises tobacco.

The at least one aerosol-former may be any suitable known compound or mixture of compounds that, in use, facilitates formation of a dense and stable aerosol and that is substantially resistant to thermal degradation at the operating temperature of the aerosol generating article. Suitable aerosol-formers are well known in the art and include, for example, polyhydric alcohols, esters of polyhydric alcohols, such as glycerol mono-, di- or triacetate, and aliphatic esters of mono-, di- or polycarboxylic acids, such as dimethyl dodecanedioate and dimethyl tetradecanedioate. Preferred aerosol formers for use in aerosol generating articles herein are polyhydric alcohols or mixtures thereof, such as triethylene glycol, 1,3-butanediol and, most preferred, glycerin.

The material capable of emitting volatile compounds in response to heating may be a charge of plant-based material. The material capable of emitting volatile compounds in response to heating may be a charge of homogenized plant-based material. For example, the aerosol generating substrate may comprise one or more materials derived from plants including, but not limited to: tobacco; tea, for example green tea; peppermint; laurel; eucalyptus; basil; sage; verbena; and tarragon. Preferably, the material capable of emitting volatile compounds in response to heating is a charge of tobacco-based material, most preferably a charge of homogenised tobacco-based material.

The aerosol generating substrate may be in the form of a plug or segment comprising a material capable of emitting volatile compounds in response to heating circumscribed by a paper or other wrapper. As stated above, where an aerosol generating substrate is in the form of such a plug or segment, the entire plug or segment including any wrapper is considered to be the aerosol generating substrate. The aerosol generating substrate preferably has a length of between about 5 mm and about 20 mm. Preferably, the aerosol generating substrate has a length of between about 6 mm and about 15 mm or a length of between about 7 mm and about 12 mm. In preferred embodiments, the aerosol generating substrate comprises a plug of tobacco-based material wrapped in a plug wrap. In particularly preferred embodiments, the aerosol generating substrate comprises a plug of homogenised tobacco-based material wrapped in a plug wrap.

Holders according to the present invention may be used with any suitable aerosol generating article.

Aerosol generating articles for use with holders according to the present invention may comprise one or more air inlets around the periphery of the aerosol generating substrate. In such embodiments, in use, cool air is drawn into the aerosol generating substrate of the aerosol generating article through the air inlets. The air drawn into the aerosol generating substrate through the air inlets passes downstream through the aerosol generating article from the aerosol generating substrate and exits the aerosol generating article through the mouthpiece or proximal end thereof.

In such embodiments, during puffing by a user the cool air drawn through the one or more air inlets around the periphery of the aerosol generating substrate advantageously reduces the temperature of the aerosol generating substrate. This advantageously substantially prevents or inhibits spikes in the temperature of the aerosol generating substrate during puffing by a user. As used herein, the term 'cool air' is used to describe ambient air that is not significantly heated by the combustible heat source upon puffing by a user.

Aerosol generating articles described herein may comprise a heat conducting element around and in direct contact with both at least a rear portion of the heat source and at least a front portion of the aerosol generating substrate. The heat conducting element provides a thermal link between the combustible heat source and the aerosol generating substrate and advantageously helps to facilitate adequate heat transfer from the combustible heat source to the aerosol generating substrate to provide an acceptable aerosol.

Suitable heat conducting elements for use herein include, but are not limited to: metal foil wrappers such as, for example, aluminum foil wrappers, steel wrappers, iron foil wrappers and copper foil wrappers; and metal alloy foil wrappers.

Aerosol generating articles described herein may comprise a mouthpiece located at the proximal end thereof. Preferably, the mouthpiece is of low filtration efficiency, more preferably of very low filtration efficiency. The mouthpiece may be a single segment or component mouthpiece. Alternatively, the mouthpiece may be a multi-segment or multi-component mouthpiece.

The mouthpiece may comprise a filter comprising one or more segments comprising suitable known filtration materials. Suitable filtration materials are known in the art and include, but are not limited to, cellulose acetate and paper. Alternatively or in addition, the mouthpiece may comprise one or more segments comprising absorbents, adsorbents, flavorants, and other aerosol modifiers and additives or combinations thereof.

Aerosol generating articles described herein preferably further comprise a transfer element or spacer element between the aerosol generating substrate and the mouthpiece. The transfer element may abut one or both of the aerosol generating substrate and the mouthpiece. Alternatively, the transfer element may be spaced apart from one or both of the aerosol generating substrate and the mouthpiece.

The inclusion of a transfer element advantageously allows cooling of the aerosol generated by heat transfer from the combustible heat source to the aerosol-generating substrate. The inclusion of a transfer element also advantageously allows the overall length of the aerosol generating article to be adjusted to a desired value, for example to a length similar to that of a conventional cigarette, through an appropriate choice of the length of the transfer element.

The transfer element may have a length of between about 7 mm and about 50 mm, for example a length of between about 10 mm and about 45 mm or of between about 15 mm and about 30 mm. The transfer element may have other lengths depending upon the desired overall length of the aerosol generating article, and the presence and length of other components within the aerosol generating article.

Preferably, the transfer element comprises at least one open-ended tubular hollow body. In such embodiments, in use, air drawn into the aerosol generating article passes through the at least one open-ended tubular hollow body as it passes downstream through the aerosol generating article from the aerosol generating substrate to the mouthpiece. The transfer element may comprise at least one open-ended tubular hollow body formed from one or more suitable materials that are substantially thermally stable at the temperature of the aerosol generated by the transfer of heat from the combustible heat source to the aerosol generating substrate. Suitable materials are known in the art and include, but are not limited to, paper, cardboard, plastics, such a cellulose acetate, ceramics and combinations thereof.

Alternatively or in addition, aerosol generating articles described herein may comprise an aerosol cooling element or heat exchanger between the aerosol generating substrate and the mouthpiece. The aerosol cooling element may comprise a plurality of longitudinally extending channels. The aerosol cooling element may comprise a gathered sheet of material selected from the group consisting of metallic foil, polymeric material, and substantially nonporous paper or cardboard. In certain embodiments, the aerosol-cooling element may comprise a gathered sheet of material selected from the group consisting of polyethylene (PE), polypropylene (PP), polyvinylchloride (PVC), polyethylene terephthalate (PET), polylactic acid (PLA), cellulose acetate (CA), and aluminum foil. Preferably the aerosol-cooling element may comprise a gathered sheet of biodegradable polymeric material, such as polylactic acid (PLA) or a grade of Mater-Bi® (a commercially available family of starch based copolyesters).

The aerosol generating articles described herein may comprise an outer wrapper that circumscribes the aerosol generating substrate and at least a rear portion of the heat source or heat source holder. The outer wrapper should grip the heat source and heat source holder and the aerosol generating substrate of the aerosol generating article when the aerosol generating article is assembled. Preferably the outer wrapper circumscribes the aerosol generating substrate, at least a rear portion of the heat source and heat source holder and any other components of the aerosol generating article downstream of the aerosol generating substrate. Outer wrappers may be formed from any suitable material or combination of materials. Suitable materials are well known in the art and include, but are not limited to, cigarette paper. Alternatively or in addition, the mouthpiece may be circumscribed by tipping paper. Aerosol generating articles described herein may be assembled using known methods and machinery.

The aerosol generating article may be substantially cylindrical in shape. The aerosol generating or may be substantially elongate. The aerosol generating or has a length and a circumference substantially perpendicular to the length. The aerosol generating substrate may be substantially cylindrical in shape. The aerosol generating substrate may be substantially elongate. The aerosol generating substrate also has a length and a circumference substantially perpendicular to the length. The aerosol generating substrate may be located in the aerosol generating or such that the length of the aerosol generating substrate is substantially parallel to the airflow direction in the aerosol generating article. The transfer section or element may be substantially elongate.

The aerosol generating article may have any desired length. For example, the aerosol generating article may have a total length of between approximately 65 mm and approximately 100 mm. The aerosol generating article may have any desired external diameter. For example, the aerosol generating article may have an external diameter of between approximately 5 mm and approximately 12 mm.

One or more of the filter, transfer element, aerosol cooling element, and heat exchanger may be incorporated into a holder of the present invention rather than being included in the aerosol generating article. The length of the aerosol generating article may be reduced if one or more of the filter, transfer element, aerosol cooling element, and heat exchanger are incorporated into the holder. In some preferred embodiments, the holder comprises one or more of the filter, transfer element, aerosol cooling element, and heat exchanger, and the holder is re-usable.

All scientific and technical terms used herein have meanings commonly used in the art unless otherwise specified. The definitions provided herein are to facilitate understanding of certain terms used frequently herein.

As used herein, the singular forms "a", "an", and "the" encompass embodiments having plural referents, unless the content clearly dictates otherwise.

As used herein, "or" is generally employed in its sense including "and/or" unless the content clearly dictates otherwise. The term "and/or" means one or all of the listed elements or a combination of any two or more of the listed elements.

As used herein, "have", "having", "include", "including", "comprise", "comprising" or the like are used in their open ended sense, and generally mean "including, but not limited to". It will be understood that "consisting essentially of", "consisting of", and the like are subsumed in "comprising," and the like.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure, including the claims.

Reference will now be made to the drawings, which depict one or more aspects described in this disclosure. However, it will be understood that other aspects not depicted in the drawing fall within the scope and spirit of this disclosure. Like numbers used in the figures refer to like components, steps and the like. However, it will be understood that the use of a number to refer to a component in a given figure is not intended to limit the component in another figure labeled with the same number. In addition, the use of different numbers to refer to components in different figures is not intended to indicate that the different numbered components cannot be the same or similar to other numbered components.
**FIG. 1** is schematic perspective view of an illustrative aerosol generating article **100** with the wrapper **110** partially opened to view the internal contents.
**FIG. 2** is a schematic sectional view of an illustrative holder **200.**
**FIG. 3** is a schematic sectional view of an illustrative aerosol generating article **100** received by an illustrative holder **200.**
**FIGS. 4A** and **4B** are schematic sectional views of an illustrative cage of a holder, where the cage includes a rotatable ring cover **300.**
**FIG. 5** is a schematic perspective view of an illustrative holder **200.**

The schematic drawings are not necessarily to scale and are presented for purposes of illustration and not limitation.

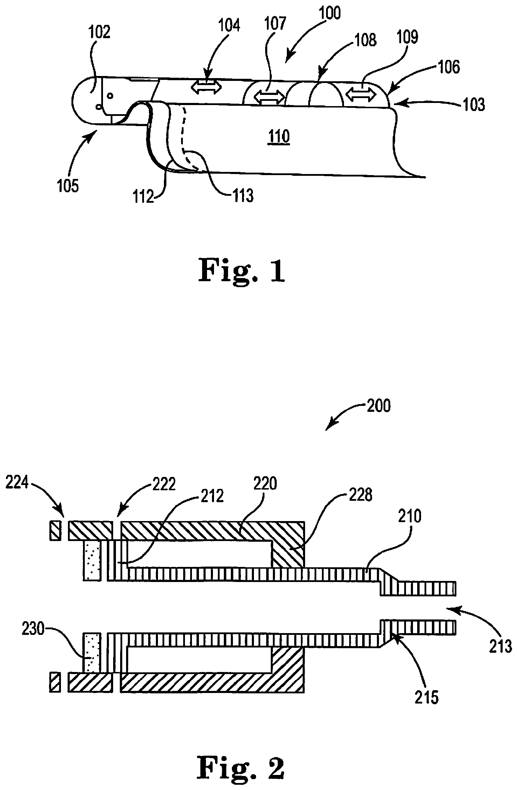

Referring now to **FIG. 1**, an aerosol generating article **100** extends between a proximal end **103** and a distal end **105.The** aerosol generating article **100** includes a combustible heat source **102** positioned at the distal end **105** of the aerosol generating article **100**, an aerosol generating substrate **104** downstream of the combustible heat source **102** and a mouthpiece **106** downstream of the aerosol generating substrate **104** and positioned at the proximal end **103** of the aerosol generating article **100**.

The aerosol generating article **100** comprises a combustible heat source **102**, an aerosol generating substrate **104**, an aerosol cooling element **107**, an elongate expansion chamber or transfer element **108** and a mouthpiece **106**, in sequential, abutting coaxial alignment, which are overwrapped in an outer wrapper **110** of, for example, cigarette paper. The combustible heat source **102** is cylindrical.

The aerosol generating substrate **104** is located immediately downstream of the combustible heat source **102** and comprises a cylindrical plug of homogenized tobacco material comprising, for example, glycerin as aerosol former and circumscribed by filter plug wrap. A heat conducting element **112**, consisting of a tube of aluminum foil, surrounds and is in contact with a rear portion of the combustible heat source **102** and an abutting front portion of the aerosol generating substrate **104**. The elongate expansion chamber **108** is located downstream of the aerosol generating substrate **104** and comprises a cylindrical open-ended tube of cardboard. The mouthpiece **106** is located downstream of the expansion chamber **108** and comprises a cylindrical plug of cellulose acetate tow **109** circumscribed by filter plug wrap.

In use, the user ignites the combustible heat source which heats the aerosol generating substrate to produce an aerosol. When the user inhales on the mouthpiece **106** air is drawn through the aerosol generating substrate **104** through air inlet holes **113** in the cigarette paper **110** and adjacent to the aerosol generating substrate **104**, through the expansion chamber **108**, through the mouthpiece **106** and into the user's mouth.

Referring now to **FIG. 2**, a holder **200** includes a mouthpiece **210** and a cage **220** slidably disposed over the mouthpiece **210**. The mouthpiece **200** defines a passage **213** that extends from a mouth end to the distal end. The passage **213** is configured to slidably receive an aerosol generating article. The passage includes a shoulder **218** or reduced inner diameter region to prevent insertion of the aerosol generating article beyond the shoulder **218**. The mouthpiece **200** also comprises a distal flange **212**.

The cage **220** includes a proximal stop **228** that engages with flange **212** when the cage **220** is slid distally over the mouthpiece **210** to prevent further distal movement of the cage **220**. The holder includes a seal **230** that extends from in inner surface of the cage **220** into the bore of the cage **220**. The seal **230** is arranged and configured to sealingly engage an aerosol generating article when the article is inserted in the holder **200**. The seal **230** may also engage with flange **212** to prevent the cage from sliding proximally off the mouthpiece **210**. The depicted cage **220** includes holes **222**, **224** through a wall of the cage downstream (**222**) and upstream (**224**) of the seal **230**. The upstream holes **224** allow air flow for lighting of a heat source, continued combustion of the heat source, and evacuation of combustion gasses. Some exchange of gas through the open distal face and radial clearance between the heat source (not shown) and the cage **220**. The downstream holes **222** allow for cool air to be drawn through the holes **222** and the aerosol generating article when the article is received in the holder **200**.

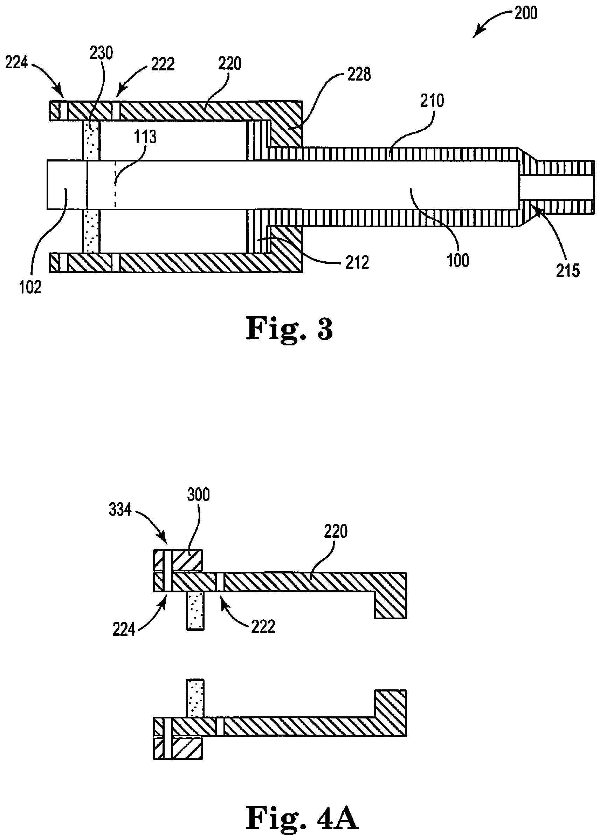

Referring now to **FIG. 3**, an aerosol generating article **100** is disposed in a holder **200**. The proximal end of the article **100** is in contact with shoulder **215** or reduced inner diameter portion of the mouthpiece **210**, indicating that article **100** is fully received within the passage of the mouthpiece **210**. The cage **220** is distally advanced over the mouthpiece **210** and stop **228** is in contact with flange **218**. The seal **230** contacts and sealingly engages the aerosol generating article **100** downstream of the heat source **102**. The distal portion of the cage **220** surrounds the heat source **102**. Openings **224**, in addition to the open distal face of the cage, allows air flow to the heat source to allow lighting, continued combustion and evacuation of combustion gasses. Openings **222** allow cool air to be drawn through the wall of the cage downstream of the seal **230**, through air inlet holes **113**, through or around aerosol generating substrate, through the remainder of the aerosol generating article **100**, through passage of mouthpiece **210** and into a user's mouth. Seal **230** prevents or limits the amount of number of combustion products from combustion of the heat source **102** from entering the cool air taken in through holes **222** downstream of the seal **230**.

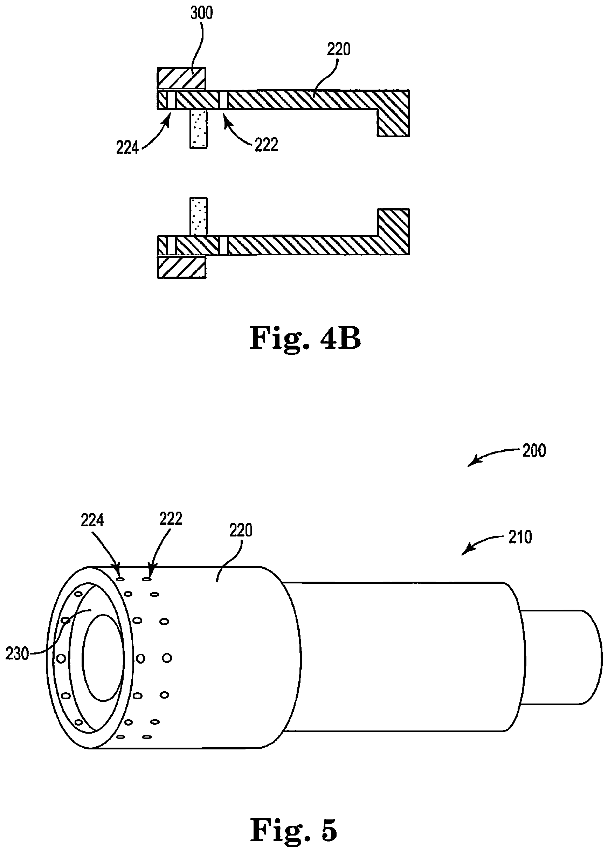

Referring now to **FIGS. 4A** and **4B**, a cage **220** includes a cover **300** for covering distal holes **224** of cage **220** for aiding in extinguishing a heat source of a smoking article (not shown). The depicted cover **300** is a ring rotatable around the longitudinal axis of the cage **220**. The cover **300** includes holes **334** through a body of the cover that may align with distal openings **224** of cage **220** (**FIG 4A**)**.** The cover **300** includes portions that when appropriately rotated (relative to **FIG. 4A**) cover distal openings **224** of cage **220** (**FIG. 4B**). When the distal openings **224** are covered, the heat source **102** may not receive sufficient air flow so that the heat source may be extinguished.

Referring now to **FIG. 5** a schematic perspective view of an illustrative holder **200** is shown. The holder **200** includes a cage **220** slidable about a mouthpiece **210** that defines a passage (not shown) configured to slidably receive an aerosol generating article. A seal **230** extends from an inner surface of the cage **220** and is configured and arranged to sealing engage the aerosol generating article downstream of a heat source. The cage **220** comprises a plurality of ventilation holes **222** downstream of the seal **230** and a plurality of ventilation holes **224** upstream of the seal **230.** The mouthpiece **210** may be telescoping so that the mouth end can be moved distally relative to a distal portion of the mouthpiece to eject an aerosol generating article.

Thus, methods, systems, apparatuses, assemblies and articles for a holder for aerosol generating articles are described. Various modifications and variations of the invention will be apparent to those skilled in the art without departing from the scope of the claims. Although the invention has been described in connection with specific preferred embodiments, it should be understood that the invention as claimed should not be unduly limited to such specific embodiments.

## Claims

1. A holder (200) for an aerosol generating article (100), comprising:
a mouthpiece (210) having a mouth end and a distal end and defining a passage (213) extending from the mouth end to the distal end, wherein the passage is configured to receive the aerosol generating article inserted in a direction from the distal end towards the mouth end;
a cage (220) extending from the mouthpiece and configured to surround at least a portion of a heat source (102) of the aerosol generating article and configured to allow air to access the heat source, wherein the cage is slidable about the mouthpiece; and
a seal (230) extending from an inner surface of the cage, wherein the seal is arranged to surround and contact the aerosol generating article downstream of the heat source,
wherein the mouthpiece defines a flange (212) and the cage defines a stop (228) configured to engage the flange as the cage is distally advanced over the mouthpiece.

2. A holder (200) according to claim 1, wherein the cage (220) comprises a tubular wall defining one or more holes (222) through the wall downstream of the seal (230).

3. A holder (200) according to claim 2, wherein the wall of the cage further defines one or more holes (224) through the wall upstream of the seal (230).

4. A holder (200) according to claim 3, further comprising a cover (300) moveable about the cage (220) between a first position and a second position, the cover comprising one or more through holes (334) that are aligned with the holes (224) of the cage (220) that are upstream of the seal (230) when the cover is in the first position, and wherein the cover covers the holes of the cage that are upstream of the seal when the cover is in the second position.

5. A holder (200) according to claim 4, wherein the cover (300) comprises a ring rotatable about a longitudinal axis of the cage (220).

6. A holder (200) according to any one of the preceding claims, wherein the passage (218) defined by the mouthpiece (210) comprises a stepped or tapered inner diameter and wherein the inner diameter in proximity to the mouth end is configured to be smaller than the outer diameter of the aerosol generating article (100) to prevent advancement of the aerosol generating article in the passage (213) beyond the mouth end.

7. An assembly comprising a holder (200) according to any preceding claim and an aerosol generating article (100) having a heat source (102), wherein the aerosol generating article is received in the passage (213) defined by the mouthpiece (210).

8. A kit comprising a holder according to any of claims 1 to 6 and one or more aerosol generating articles (100) having a heat source (102), wherein the aerosol generating articles are configured to be received by the passage of the mouthpiece.

9. An assembly according to claim 7 or a kit according to claim 8, wherein the aerosol generating article (100) comprises an aerosol generating substrate (104) and wherein the aerosol generating article is configured to transfer heat from the heat source (102) to the aerosol generating substrate without combusting the aerosol generating substrate.

10. An assembly or kit according to claim 9, wherein the aerosol generating substrate (104) comprises tobacco.

11. An assembly or kit according to claim 9 or claim 10,
wherein the aerosol generating article (100) further comprises a wrapper (110) circumscribing the aerosol generating substrate (104), the wrapper defining one or more ventilation through holes (113),
wherein the cage (220) comprises a tubular wall defining one or more holes (222) through the wall downstream of the seal (230);
wherein the assembly is configured such that drawing on the mouthpiece (110) causes air to flow through the one or more holes of the tubular wall of the cage and through the one or more holes of the wrapper of the aerosol generating article.

## Patentansprüche

1. Halter (200) für einen aerosolerzeugenden Artikel (100), aufweisend:
ein Mundstück (210) mit einem Mundende und einem distalen Ende, das einen Durchgang (213) definiert, der sich vom Mundende zum distalen Ende erstreckt, wobei der Durchgang ausgelegt ist, den aerosolerzeugenden Artikel aufzunehmen, der in einer Richtung vom distalen Ende zum Mundende eingesetzt wird;
einen Käfig (220), der sich vom Mundstück erstreckt und derart ausgelegt ist, dass er mindestens einen Abschnitt einer Wärmequelle (102) des aerosolerzeugenden Artikels umgibt, und ausgelegt ist, zu ermöglichen, dass Luft an die Wärmequelle gelangt, wobei der Käfig über das Mundstück verschiebbar ist; und
eine Dichtung (230), die sich von einer Innenfläche des Käfigs erstreckt, wobei die Dichtung derart ausgeführt ist, dass sie den aerosolerzeugenden Artikel nachgeschaltet der Wärmequelle umgibt und kontaktiert,
wobei das Mundstück einen Flansch (212) definiert und der Käfig einen Anschlag (228) definiert, der ausgelegt ist, in den Flansch einzugreifen, während der Käfig distal über das Mundstück vorgeschoben wird.

2. Halter (200) nach Anspruch 1, wobei der Käfig (220) eine röhrenförmige Wand aufweist, die nachgeschaltet der Dichtung (230) ein oder mehrere Löcher (222) durch die Wand definiert.

3. Halter (200) nach Anspruch 2, wobei die Wand des Käfigs zuströmseitig der Dichtung (230) weiter ein oder mehrere Löcher (224) durch die Wand definiert.

4. Halter (200) nach Anspruch 3, weiter aufweisend eine Abdeckung (300), die zwischen einer ersten Stellung und einer zweiten Stellung um den Käfig (220) beweglich ist, wobei die Abdeckung ein oder mehrere Durchgangslöcher (334) aufweist, die mit den Löchern (224) des Käfigs (220) ausgerichtet sind, die sich zuströmseitig der Dichtung (230) befinden, wenn sich die Abdeckung in der ersten Stellung befindet, und wobei die Abdeckung die Löcher des Käfigs abdeckt, die sich zuströmseitig von der Dichtung befinden, wenn sich die Abdeckung in der zweiten Stellung befindet.

5. Halter (200) nach Anspruch 4, wobei die Abdeckung (300) einen Ring aufweist, der um eine Längsachse des Käfigs (220) drehbar ist.

6. Halter (200) nach einem der vorstehenden Ansprüche, wobei der Durchgang (218), der durch das Mundstück (210) definiert ist, einen stufenförmigen oder kegelförmigen Innendurchmesser aufweist, und wobei der Innendurchmesser in der Nähe des Mundendes derart ausgelegt ist, dass er kleiner als der Außendurchmesser des aerosolerzeugenden Artikels (100) ist, um ein Vorrücken des aerosolerzeugenden Artikels in den Durchgang (213) über das Mundende hinaus zu verhindern.

7. Anordnung, die einen Halter (200) nach einem der vorstehenden Ansprüche und einen aerosolerzeugende Artikel (100) mit einer Wärmequelle (102) aufweist, wobei der aerosolerzeugende Artikel in dem durch das Mundstück (210) definierten Durchgang (213) aufgenommen ist.

8. Kit, das einen Halter nach einem der Ansprüche 1 bis 6 und ein oder mehrere aerosolerzeugende Artikel (100) mit einer Wärmequelle (102) aufweist, wobei die aerosolerzeugenden Artikel ausgelegt sind, von dem Durchgang des Mundstücks aufgenommen zu werden.

9. Anordnung nach Anspruch 7 oder Kit nach Anspruch 8, wobei der aerosolerzeugende Artikel (100) ein aerosolerzeugendes Substrat (104) aufweist, und wobei der aerosolerzeugende Artikel ausgelegt ist, Wärme von der Wärmequelle (102) auf das aerosolerzeugende Substrat zu übertragen, ohne das aerosolerzeugende Substrat zu verbrennen.

10. Anordnung oder Kit nach Anspruch 9, wobei das aerosolerzeugende Substrat (104) Tabak aufweist.

11. Anordnung oder Kit nach Anspruch 9 oder Anspruch 10,
wobei der aerosolerzeugende Artikel (100) weiter eine Umhüllung (110) aufweist, die das aerosolerzeugende Substrat (104) abgrenzt, und die Umhüllung ein oder mehrere Belüftungsdurchgangslöcher (113) definiert,
wobei der Käfig (220) eine röhrenförmige Wand aufweist, die nachgeschaltet der Dichtung (230) ein oder mehrere Löcher (222) durch die Wand definiert;
wobei die Anordnung derart ausgelegt ist, dass ein Ziehen an dem Mundstück (110) bewirkt, dass Luft durch das eine oder die mehreren Löcher der röhrenförmigen Wand des Käfigs und durch das eine oder die mehreren Löcher der Umhüllung des aerosolerzeugenden Artikels strömt.

## Revendications

1. Un récipient (200) pour un article de génération d'aérosol (100), comprenant :
un embout buccal (210) ayant une extrémité buccale et une extrémité distale et définissant un passage (213) s'étendant de l'extrémité buccale à l'extrémité distale, où le passage est configuré pour recevoir l'article de génération d'aérosol inséré dans une direction de l'extrémité distale vers l'extrémité buccale ;
une cage (220) s'étendant depuis l'embout buccal et configurée pour entourer au moins une partie d'une source de chaleur (102) de l'article de génération d'aérosol et configurée pour permettre à l'air d'accéder à la source de chaleur, où la cage peut glisser autour de l'embout buccal ; et
un joint (230) s'étendant d'une surface intérieure de la cage, où le joint est agencé pour entourer et pour entrer en contacte avec l'article de génération d'aérosol en aval de la source de chaleur,
dans lequel l'embout buccal définit une bride (212) et la cage définit un arrêt (228) configuré pour engager la bride lorsque la cage est avancée distalement sur l'embout buccal.

2. Support (200) selon la revendication 1, dans lequel la cage (220) comprend une paroi tubulaire définissant un ou plusieurs trous (222) à travers la paroi en aval du joint (230).

3. Support (200) selon la revendication 2, dans lequel la paroi de la cage définit en outre un ou plusieurs trous (224) à travers la paroi en amont du joint (230).

4. Support (200) selon la revendication 3, comprenant en outre un couvercle (300) mobile sur la cage (220) entre une première position et une deuxième position, le couvercle comprenant un ou plusieurs trous traversantes (334) qui sont alignés avec les trous (224) de la cage (220) qui sont en amont du joint (230) lorsque le couvercle est dans la première position, et dans lequel le couvercle couvre les trous de la cage qui sont en amont du joint lorsque le couvercle est dans la deuxième position.

5. Support (200) selon la revendication 4, dans lequel le couvercle (300) comprend une bague pouvant tourner autour d'un axe longitudinal de la cage (220).

6. Support (200) selon l'une quelconque des revendications précédentes, dans lequel le passage (218) défini par l'embout buccal (210) comprend un diamètre intérieur étagé ou effilé et, dans lequel le diamètre intérieur à proximité de l'extrémité buccale est configuré pour être plus petit que le diamètre extérieur de l'article de génération d'aérosol (100) pour empêcher l'avancement de l'article de génération d'aérosol dans le passage (213) au-delà de l'extrémité buccale.

7. Ensemble comprenant un support (200) selon l'une quelconque des revendications précédentes et un article de génération d'aérosol (100) ayant une source de chaleur (102), dans lequel l'article de génération d'aérosol est reçu dans le passage (213) défini par l'embout buccale (210).

8. Kit comprenant un support selon l'une quelconque des revendications 1 à 6 et un ou plusieurs articles de génération d'aérosol (100) ayant une source de chaleur (102), dans lequel les articles de génération d'aérosol sont configurés pour être reçus dans le passage de l'embout buccale.

9. Ensemble selon la revendication 7 ou kit selon la revendication 8, dans lequel l'article de génération d'aérosol (100) comprend un substrat de génération d'aérosol (104) et dans lequel l'article de génération d'aérosol est configuré pour transférer la chaleur de la source de chaleur (102) vers le substrat de génération d'aérosol sans brûler le substrat de génération d'aérosol.

10. Ensemble ou kit selon la revendication 9, dans lequel le substrat de génération d'aérosol (104) comprend du tabac.

11. Ensemble ou kit selon la revendication 9 ou la revendication 10,
dans lequel l'article de génération d'aérosol (100) comprend en outre une enveloppe (110) entourant le substrat de génération d'aérosol (104), l'enveloppe définissant un ou plusieurs trous traversants de ventilation (113),
dans lequel la cage (220) comprend une paroi tubulaire définissant un ou plusieurs trous (222) à travers la paroi en aval du joint (230) ;
dans lequel l'ensemble est configuré de telle sorte que le fait d'aspirer par l'embout buccal (110) provoque l'écoulement d'air par l'un ou plusieurs trous de la paroi tubulaire de la cage et à travers l'un ou plusieurs trous de l'enveloppe de l'article de génération d'aérosol.
